# EUROPEAN PATENT APPLICATION

(11) **EP 4 233 933 A1**
(43) Date of publication of application: **30.08.2023**
(21) Application number: 21882435.7
(22) Date of filing: 23.08.2021
(51) Int. Cl.: A61M 1/36, A61M 1/18

(54) **EXTRACORPOREAL CIRCULATION CIRCUIT, FLUID PROCESSING DEVICE, CONNECTION STRUCTURE, AND PROCESSING METHOD FOR FLUID PROCESSING DEVICE IN EXTRACORPOREAL CIRCULATION CIRCUIT**

(30) Priority: 21.10.2020 JP 2020176603
(71) Applicant: JMS Co., Ltd., Hiroshima-shi Hiroshima 730-8652 (JP)
(72) Inventor: IZUMI Ryohei, Hiroshima-shi, Hiroshima 730-8652 (JP); INOUE Keisuke, Hiroshima-shi, Hiroshima 730-8652 (JP); YAMAMOTO Mutsumi, Hiroshima-shi, Hiroshima 730-8652 (JP)
(74) Representative: HGF
(86) International application number: PCT/JP2021/030796
(87) International publication number: WO 2022/085294

(57) **Abstract**

Provided are: a fluid processing device that can easily be attached/detached or replaced; a connection structure; and a processing method for the fluid processing device in an extracorporeal circulation circuit. An extracorporeal circulation circuit 100 according to the present invention comprises: a first tube 101D in which a tube-side first attachment/detachment part 40B is provided at an end section; a second tube 101U in which a tube-side second attachment/detachment part 50A, which can be attached to/detached from the tube-side first attachment/detachment part 40B, is provided at an end section; and a fluid processing device 1 in which a device-side first attachment/detachment part 40A that can be attached to/detached from the tube-side second attachment/detachment part 50A is provided to one of a fluid inlet 25 and a fluid outlet 28, and a device-side second attachment/detachment part 50B that can be attached to/detached from the tube-side first attachment/detachment part 40B is provided to the other of the fluid inlet 25 and the fluid outlet 28.

## Description

### TECHNICAL FIELD

The present invention relates to an extracorporeal circulation circuit, a fluid processing device, a connection structure, and a processing method for a fluid processing device in an extracorporeal circulation circuit.

### BACKGROUND ART

Conventionally, in heart surgery and the like, an extracorporeal circulation circuit, which is a blood circulation circuit provided with an artificial lung, has been used for taking over extracorporeal circulation functions of a patient undergoing the surgery.

When such an extracorporeal circulation circuit is to be used, to prevent mixing of unwanted substances and air into the blood during operation, a priming process is performed in advance by causing a priming fluid such as saline to circulate in the extracorporeal circulation circuit (see Patent Document 1). In the extracorporeal circulation circuit, a fluid processing device is provided, which is a cleaning-purpose filter device to capture unwanted substances at the time of the priming process. The fluid processing device is removed from the extracorporeal circulation circuit after the priming process is performed.

In a case of using an extracorporeal circulation circuit provided with an artificial lung serving as a fluid processing device, the artificial lung may become clogged. In such a situation, the clogged artificial lung needs to be replaced with a new artificial lung, or the entire blood circuit including not only the artificial lung but also the fluid processing device needs to be replaced, which will both result in an increase in costs.

Patent Document 1: Japanese Unexamined Patent Application, Publication No. 2008-246141

### DISCLOSURE OF THE INVENTION

### Problems to be Solved by the Invention

However, there is a disadvantage that removal or replacement of the fluid processing device is a difficult work and requires time and effort. In addition, when a fluid processing device has trouble such as clogging, the entirety of an extracorporeal circulation system including circuits is replaced in many situations, which will disadvantageously incur an extra cost in comparison with discarding a part of the system.

It is an object of the present invention to provide an extracorporeal circulation circuit, a fluid processing device, a connection structure, and a processing method for a fluid processing device in an extracorporeal circulation circuit that make it possible to easily remove or replace the fluid processing device.

### Means for Solving the Problems

To achieve the above object, the present invention provides an extracorporeal circulation circuit including: a first tube having a tube-side first attachment/detachment part provided at an end portion thereof; a second tube having a tube-side second attachment/detachment part provided at an end portion thereof, the tube-side second attachment/detachment part being attachable to and detachable from the tube-side first attachment/detachment part; and a fluid processing device having a fluid inlet and a fluid outlet, and including a device-side first attachment/detachment part that is provided at one of the fluid inlet and the fluid outlet and that is attachable to and detachable from the tube-side second attachment/detachment part, and a device-side second attachment/detachment part that is provided at an other of the fluid inlet and the fluid outlet and that is attachable to and detachable from the tube-side first attachment/detachment part.

The extracorporeal circulation circuit may further include a lock mechanism capable of locking and unlocking the tube-side first attachment/detachment part or the device-side first attachment/detachment part to and from the tube-side second attachment/detachment part or the device-side second attachment/detachment part.

The fluid processing device may be a filter device.

The fluid processing device may be disposed at a time of priming.

To achieve the above object, the present invention provides a connection structure including: a tube-side first attachment/detachment part provided at an end portion of a first tube; a tube-side second attachment/detachment part that is provided at an end portion of a second tube and that is attachable to and detachable from the tube-side first attachment/detachment part; a device-side first attachment/detachment part that is provided at one of a fluid inlet and a fluid outlet of a fluid processing device and that is attachable to and detachable from the tube-side second attachment/detachment part; and a device-side second attachment/detachment part that is provided at an other of the fluid inlet and the fluid outlet of the fluid processing device and that is attachable to and detachable from the tube-side first attachment/detachment part.

The connection structure may further include a lock mechanism capable of locking and unlocking the tube-side first attachment/detachment part or the device-side first attachment/detachment part to and from the tube-side second attachment/detachment part or the device-side second attachment/detachment part.

Furthermore, to achieve the above object, the present invention provides a processing method for a fluid processing device in an extracorporeal circulation circuit including: a first tube having a tube-side first attachment/detachment part provided at an end portion thereof, a second tube having a tube-side second attachment/detachment part provided at an end portion thereof, the tube-side second attachment/detachment part being attachable to and detachable from the tube-side first attachment/detachment part, and a fluid processing device having, at a fluid inlet, a device-side second attachment/detachment part attachable to and detachable from the tube-side first attachment/detachment part and having, at a fluid outlet, a device-side first attachment/detachment part attachable to and detachable from the tube-side second attachment/detachment part. The processing method includes: a fluid circulation step that is performed in a state in which the device-side second attachment/detachment part is connected to the tube-side first attachment/detachment part and the device-side first attachment/detachment part is connected to the tube-side second attachment/detachment part and includes causing a fluid to flow into the extracorporeal circulation circuit to thereby cause the fluid to flow into the fluid processing device through the first tube and to flow out to the second tube; a detachment step that is performed after the fluid circulation step and includes detaching the tube-side first attachment/detachment part from the device-side second attachment/detachment part and detaching the tube-side second attachment/detachment part from the device-side first attachment/detachment part; and a step of connecting the tube-side first attachment/detachment part to the tube-side second attachment/detachment part or a step of replacing the fluid processing device with a new fluid processing device.

### Effects of the Invention

According to the present invention, it is possible to provide the extracorporeal circulation circuit, the fluid processing device, the connection structure, and the processing method for a fluid processing device in an extracorporeal circulation circuit that make it possible to easily remove or replace the fluid processing device.

### BRIEF DESCRIPTION OF THE DRAWINGS

FIG. 1 is a drawing for explaining a configuration of a blood circulation circuit 100;
FIG. 2 is a cross-sectional view including a cleaning filter device 1 and parts of tubes 101 connected to the cleaning filter device 1;
FIGS. 3A-3D are drawings for explaining a method for producing a filter member 10;
FIG. 4 is an exploded perspective view of the cleaning filter device 1;
FIG. 5 is a partial enlarged view of FIG. 2;
FIG. 6 is a perspective view of a second container 20B as viewed from an inner face side;
FIG. 7 is a drawing indicating a state in which an operation member 43 has been disassembled from a device-side first attachment/detachment part 40A;
FIGS. 8A-8D are drawings for explaining a procedure of attaching a tube-side second attachment/detachment part 50A of a second attachment/detachment member 80 to the device-side first attachment/detachment part 40A of the cleaning filter device 1;
FIGS. 9A-9C are drawings for explaining a procedure of removing the cleaning filter device 1; and
FIGS. 10A-10B are drawings for explaining a procedure of detaching the tube-side second attachment/detachment part 50A from the device-side first attachment/detachment part 40A.

### PREFERRED MODE FOR CARRYING OUT THE INVENTION

The following will describe embodiments of the present invention, with reference to the drawings. In an embodiment, as a fluid processing device, a cleaning filter device 1 in which a priming fluid flows will be described as an example. Further, as an extracorporeal circulation circuit, a blood circulation circuit 100 will be described as an example.

It should be noted, however, that the fluid flowing in the fluid processing device is not limited to priming fluid and may be blood, other preparations, or the like. The fluid processing device may be an artificial lung, a blood filter, a heat exchanger, a hemoconcentrator, a blood reservoir, a blood pump, and/or the like. The extracorporeal circulation circuit may be a cardioplegia circuit or the like.

### (Blood circulation circuit 100)

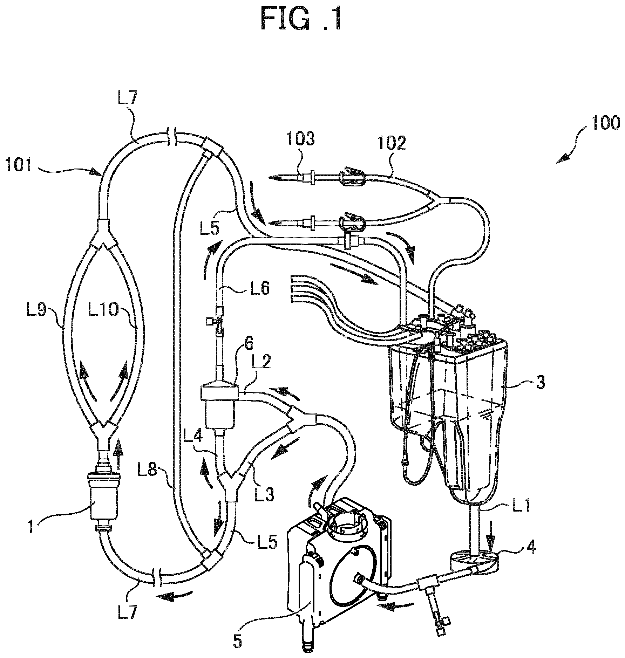

FIG. 1 is a drawing for explaining a configuration of the blood circulation circuit 100. The blood circulation circuit 100 is used in an artificial cardiopulmonary system and includes a blood reservoir 3, a blood pump 4, an artificial lung 5, a blood filter device 6, and the cleaning filter device 1 for the purpose of cleaning the circuit. These devices are connected together by tubes 101 that are flexible and made of polyvinyl chloride or the like.

In the following description, when the blood circulation circuit 100 is used as an artificial cardiopulmonary system that draws blood from and feeds blood to a patient, the use will be referred to as "normal use". Further, prior to the "normal use", an operation performed to eliminate unwanted substances and air bubbles that are present in the blood circulation circuit 100 by causing a priming fluid (e.g., saline) to circulate in the blood circulation circuit 100 will be referred to as "priming".

### (Blood reservoir 3)

The blood reservoir 3 stores therein the blood drawn from the patient during the normal use and the priming fluid at the time of the priming.

### (Blood pump 4)

The blood pump 4 is disposed on the downstream side of the blood reservoir 3 and causes the blood or the priming fluid to circulate. As the blood pump 4, a known centrifugal or roller pump may be used. In an embodiment, a centrifugal pump is used for applying pressure to the blood or the priming fluid by driving a rotating body provided inside so as to cause the blood or the priming fluid to circulate in the blood circulation circuit 100.

### (Artificial lung 5)

The artificial lung 5 is disposed on the downstream side of the blood pump 4. During the normal use, the artificial lung 5 adjusts temperature of the blood fed by the blood pump 4 from the blood reservoir 3 to the patient and performs a gas exchange process (by adding oxygen, eliminating carbon dioxide, and the like) on the blood. Supplying oxygen is realized with an oxygen tank or the like, for example, so as to supply the oxygen to be added to the blood.

### (Blood filter device 6)

During the normal use, the blood filter device 6 filters the blood and eliminates air bubbles in the blood.

### (Cleaning filter device 1)

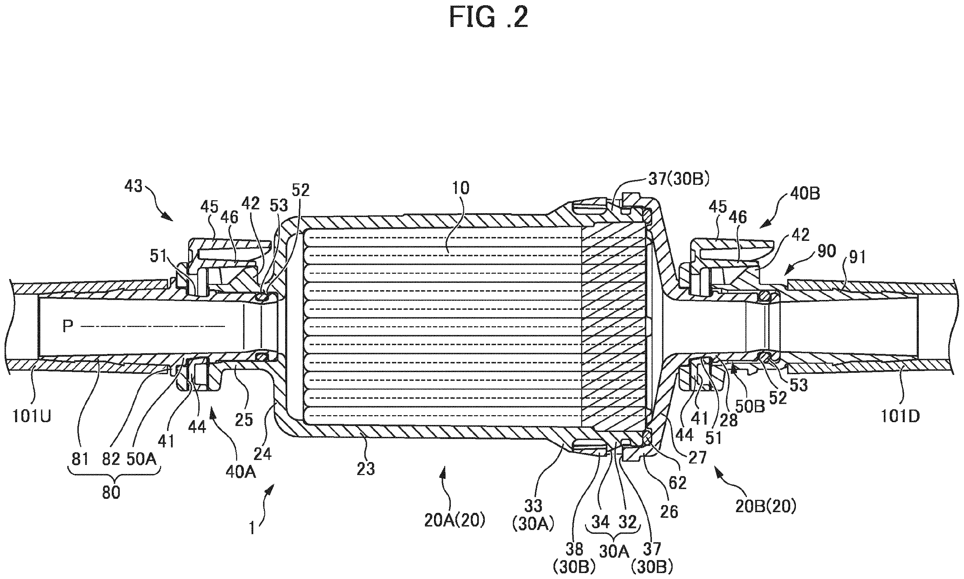

The cleaning filter device 1 is used at the time of priming the blood circulation circuit 100 and is removed after the priming. FIG. 2 is a cross-sectional view including the cleaning filter device 1 and parts of the tubes 101 connected to the cleaning filter device 1. The cleaning filter device 1 includes a filter member 10 and a container 20 housing therein the filter member 10.

### (Filter member 10)

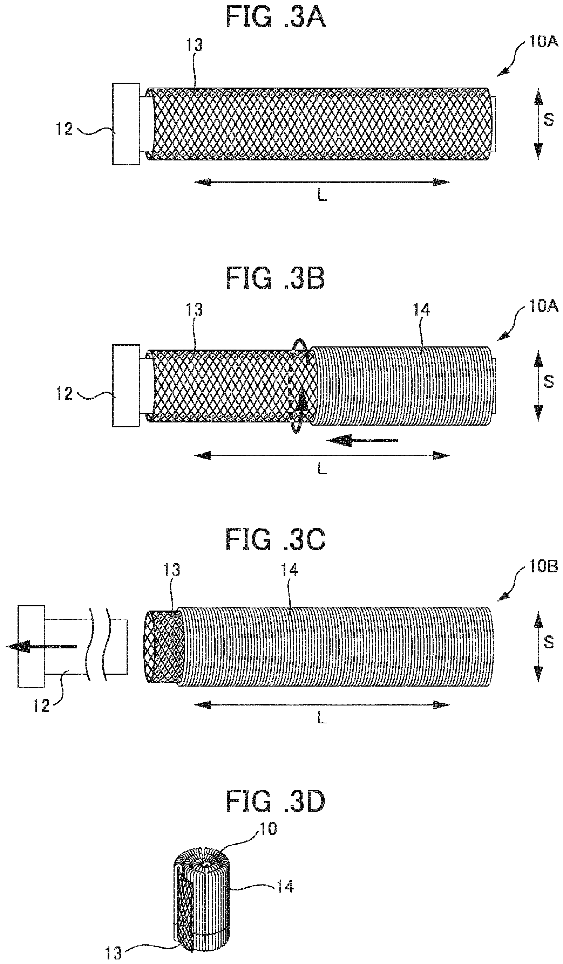

FIGS. 3A-3D are drawings for explaining a method for producing the filter member 10. At first, as shown in FIG. 3A, by placing a mesh-like spacer 13 over a core member 12 having a slender plate-like shape, a supported spacer 10A supported by the core member 12 is formed. The mesh-like spacer 13 is produced by using a polyolefin polymer material. As the spacer 13, it is preferable to use a mesh using fibers of polypropylene (PP) or polyethylene terephthalate (PET), in particular. The opening areas (pore diameters) of the mesh are in the range of 1.5 mm to 2.5 mm. The diameters of the fibers being used are in the range of 0.4 mm to 0.6 mm.

In an embodiment, the spacer 13 is supported by the core member 12, as a result of inserting the core member 12 inside the spacer 13 having a bag-like (tubular) shape. However, possible embodiments are not limited to this example. It is also acceptable to wrap the front face and the rear face of the core member 12 with the spacer 13 having a planar shape. Further, it is also acceptable to place the spacer 13 only on one of the faces of the core member 12.

Subsequently, as shown in FIG. 3B, hollow yarn 14 that is porous is wound around the outer circumference of the supported spacer 10A.

In this situation, the hollow yarn 14 is wound around so as to extend in a short direction S of the supported spacer 10A. In other words, the hollow yarn 14 is wound around so that the yarn runs from one end to the other in the short direction S on one face of the supported spacer 10A, makes a turn at the other end of the short direction S, runs from the other end to the one end of the short direction on the other face of the supported spacer 10A, and makes a turn at the one end of the short direction. This process is repeated so as to wind the hollow yarn 14 around the outer circumference of the supported spacer 10A. In this situation, it is preferable to wind the hollow yarn 14 around so that adjacently-positioned windings of the yarn do not overlap each other, while no gap is formed.

Further, the process of winding the hollow yarn 14 is carried out from one end to the other in a long direction L of the supported spacer 10A, so that the hollow yarn 14 is wound around substantially the entirety of the supported spacer 10A.

After that, as shown in FIG. 3C, the core member 12 is pulled out from one end, in the long direction L, of the supported spacer 10A on which the hollow yarn 14 has been wound around, so as to form a hollow yarn wound body 10B in which the hollow yarn 14 is wound around the outer circumference of the spacer 13.

After that, as shown in FIG. 3D, the hollow yarn wound body 10B is rolled from one end to the other in the long direction L, so as to form the filter member 10 rolled up in a circular columnar shape.

In the filter member 10 produced in this manner, because the hollow yarn 14 is wound around the spacer 13 so that the windings do not overlap each other while no gap is formed, the hollow yarn 14 is arranged uniformly on the surface of the spacer 13. As a result, because the hollow yarn 14 is arranged in a balanced manner even while in the rolled-up circular columnar shape, no unevenness occurs in the filtering.

Because the hollow yarn 14 is wound around the spacer 13, even after the core member 12 is removed and the rolled-up circular columnar shape is formed, the hollow yarn 14 keeps being arranged in an orderly formation. Thus, no unevenness occurs in the filtering. Further, because the hollow yarn 14 is wound around the spacer 13, the windings of the hollow yarn 14 are prevented from being in close contact with one another.

Further, because the spacer 13 is formed as the mesh, it is also possible to inhibit pressure loss at the time of the filtering. Furthermore, it is possible to save the hollow yarn 14 in an amount corresponding to the spacer 13.

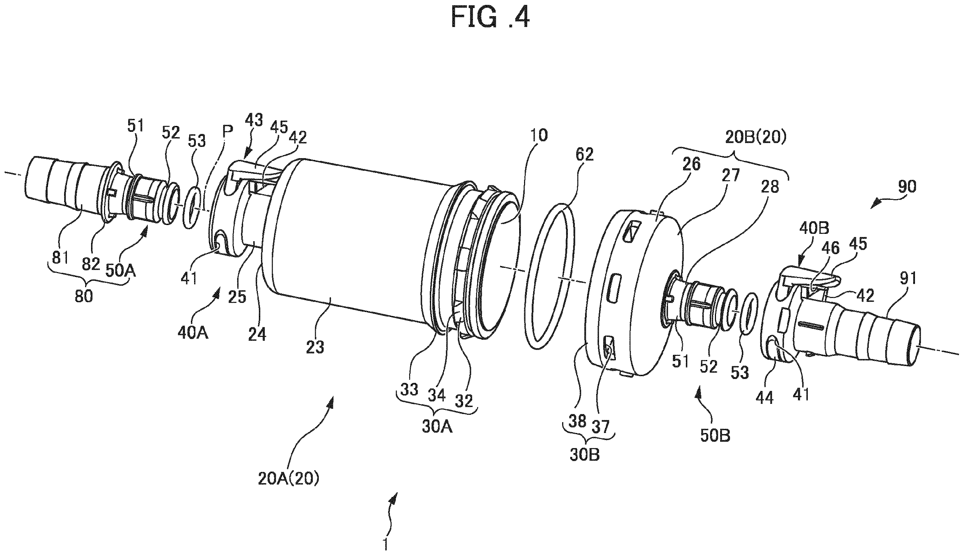

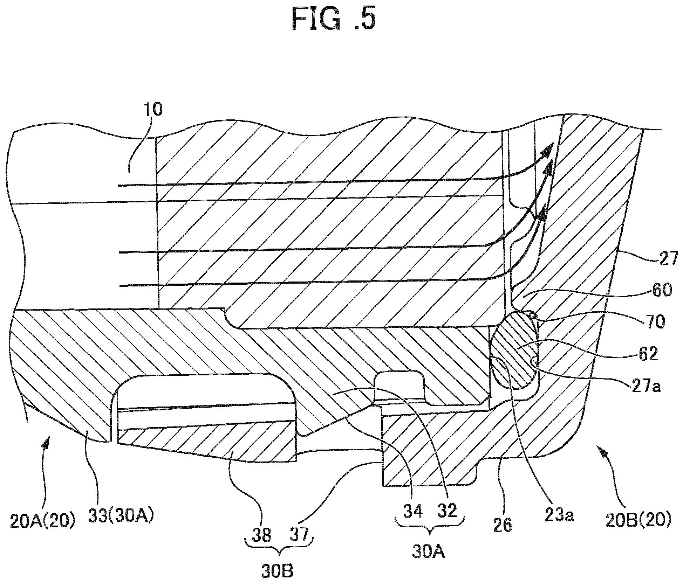

FIG. 4 is an exploded perspective view of the cleaning filter device 1. FIG. 5 is a partial enlarged view of FIG. 2.
The container 20 includes a first container 20A disposed on the upstream side (the inlet side) of the fluid and a second container 20B disposed on the downstream side (the outlet side) of the fluid.

### (First container 20A)

The first container 20A includes: a first cylinder part 23 having a cylindrical shape; a first end face part 24 having a disc shape and covering the inlet side being one of the axial end portions of the first cylinder part 23; and a fluid inlet (a first fluid inlet/outlet) 25 extending in the axial direction from a central part of the first end face part 24 toward the upstream side.

### (First link part 30A)

A first link part 30A is provided on the outer face on the downstream side being the other end portion of the first cylinder part 23.

The first link part 30A is a part linked to a second link part 30B of the second container 20B (to be described later). From the downstream side, the first link part 30A includes: engagement protrusions 32 that are provided, in an embodiment, in eight locations equally spaced in the circumferential direction; and a ring-shaped protrusion 33 that contiguously extends in the circumferential direction and is provided on the upstream side relative to the engagement protrusions 32. Note that the quantity of the engagement protrusions 32 is not limited to eight and may be larger or smaller than eight. Further, the ring-shaped protrusion 33 does not necessarily have to be contiguous in the circumferential direction and may be spatially intermittent.

With regard to each of the engagement protrusions 32, the protrusion amount thereof from the outer face of the first cylinder part 23 increases from the downstream side toward the upstream side. In other words, on the outer face of each of the engagement protrusions 32, a slope 34 ascending from the downstream side toward the upstream side is formed.

### (Second container 20B)

The second container 20B includes: a second cylinder part 26 having a circular cylindrical shape; a second end face part 27 having a disc shape and covering the outlet side being the other axial end portion of the second cylinder part 26; and a fluid outlet (a second fluid inlet/outlet) 28 extending in the axial direction from a central part of the second end face part 27 toward the downstream side.

### (Second link part 30B)

The second link part 30B is a part provided at one of the end portions of the second cylinder part 26 so as to be linked to the first container 20A.

The second link part 30B includes: engagement holes 37 that are provided in eight locations equally spaced in the circumferential direction so as to correspond to the engagement protrusions 32 of the first cylinder part 23; and a tip end part 38 provided on the upstream side relative to the engagement holes 37. Similarly to the engagement protrusions 32, the quantity of the engagement holes 37 is not limited to eight, either.

### (Ridge part)

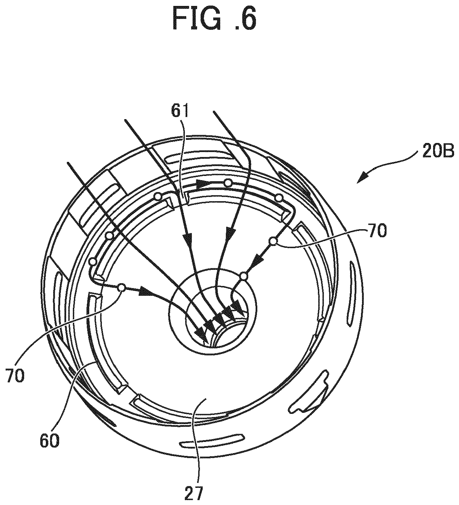

FIG. 6 is a perspective view of the second container 20B as viewed from the inner face side. On the outer circumferential side of the second end face part 27 of the second container 20B on the inner face side, a ridge part 60 extending along the circumference and formed to be non-contiguous by slits 61 is provided. The slits 61 are provided in six locations equally spaced in the circumferential direction. However, the quantity of the locations is not limited to six and may be larger or smaller than six.

### (O-ring)

On the outer circumference of the ridge part 60, as shown in FIG. 2 and FIG. 5, an O-ring 62 is provided so as to span across (not shown in FIG. 6).

As described above, the filter member 10 obtained by winding the hollow yarn 14 around the spacer 13 is, at first, housed in the first container 20A. After that, an end portion of the filter member 10 on the downstream side is adhered by using a urethane adhesive agent, so as to be fixed onto the first container 20A. As a method for the fixation, it is possible to use a centrifugal potting method, for example. Further, the second container 20B is placed over the opening part of the first container 20A on the downstream side, so as to link the first link part 30A to the second link part 30B.

In this situation, the linkage between the first link part 30A and the second link part 30B is achieved in the following manner.

When the second cylinder part 26 is plugged around the outer circumference of the first cylinder part 23, the diameter of the tip end part 38 of the second cylinder part 26 is enlarged along the slopes 34 of the engagement protrusions 32. Further, when the tip end part 38 of the second cylinder part 26 has gone over the slopes 34 of the engagement protrusions 32, the tip end part 38 of the second cylinder part 26 is fitted in a recess between the ring-shaped protrusion 33 and the engagement protrusions 32. As a result, the first link part 30A and the second link part 30B are linked together, so that the filter member 10 is housed in the interior space inside the container 20.

In this situation, as shown in FIG. 2 and FIG. 5, when the first container 20A and the second container 20B are linked together, the O-ring 62 is pressed by an end face 23a of the first cylinder part 23 and an inner face 27a of a second lateral end part, so as to be in close contact with the end face 23a and the inner face 27a. As a result, the interior space formed by the first container 20A and the second container 20B is kept airtight (fluid-tight) from the exterior space.

### (Device-side first attachment/detachment part 40A)

As shown in FIG. 2 and FIG. 4, a device-side first attachment/detachment part 40A is formed for the fluid inlet 25 that has a circular cylindrical shape with a diameter smaller than that of the first cylinder part 23 and that extends from a central part of the first end face part 24 of the first container 20A toward the upstream side, which is one of two sides. The device-side first attachment/detachment part 40A includes: a slot part 41 provided at the tip end of the fluid inlet 25; an operation protrusion 42 provided so as to protrude radially outward on the outer face of the fluid inlet 25 in a position closer to the first end face part 24 relative to the slot part 41; and an operation member 43 inserted in the slot part 41 and capable of moving in a radial direction orthogonal to an axial direction P of the device-side first attachment/detachment part 40A.

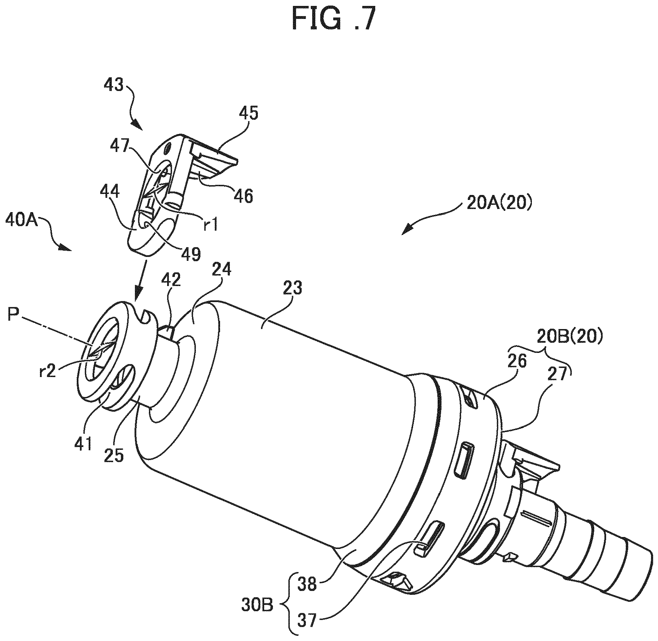

FIG. 7 is a drawing indicating a state in which the operation member 43 has been disassembled from the device-side first attachment/detachment part 40A. The operation member 43 includes: an plug part 44 plugged into the slot part 41; a pressing part 45 that extends from a part of the outer circumference of the plug part 44 in a direction (the axial direction P of the first cylinder part 23) orthogonal to the plug part 44; and an urging part 46 that extends from a section of the plug part 44 positioned radially inside the pressing part 45, so as to extend substantially parallel to the plug part 44 at a certain distance from the plug part 44.

The plug part 44 has formed therein a through hole 47 having a substantially elliptical shape. The minor diameter r1 of the through hole 47 is substantially equal to the inside diameter r2 of the fluid inlet 25.

When the operation member 43 is inserted in the slot part 41, the urging part 46 abuts against the operation protrusion 42. In that situation, of the inner face of the through hole 47 formed in the plug part 44, a face in the major diameter direction positioned opposite from the side on which the urging part 46 is provided serves as a lock face 49 that locks the second attachment/detachment member 80 (to be described later).

### (Device-side second attachment/detachment part 50B)

As shown in FIG. 4, a device-side second attachment/detachment part 50B is formed for the fluid outlet 28 that has a circular cylindrical shape with a diameter smaller than that of the second cylinder part 26 and that extends from a central part of the second end face part 27 of the second container 20B toward the downstream side, which is the other of the two sides. The device-side second attachment/detachment part 50B includes: a first circumferential groove 51 provided on the outer circumference of the fluid outlet 28 on the basal end side; and a second circumferential groove 52 provided on the outer circumference of the fluid outlet 28 on the tip end side. An O-ring 53 is attached to the second circumferential groove 52.

### (Second attachment/detachment member 80)

As shown in FIG. 2, an upstream-side tube 101U is connected to the device-side first attachment/detachment part 40A positioned on the inlet side of the cleaning filter device 1. To the upstream-side tube 101U, the second attachment/detachment member 80 is attached, while being formed with a tube-side second attachment/detachment part 50A having the same structure as the device-side second attachment/detachment part 50B positioned on the outlet side of the cleaning filter device 1.

The second attachment/detachment member 80 includes: an insertion part 81 inserted in the upstream-side tube 101U; an abutment part 82 that abuts against a tip end of the upstream-side tube 101U; and the tube-side second attachment/detachment part 50A extending from the abutment part 82 to the outside of the upstream-side tube 101U.

The tube-side second attachment/detachment part 50A has an outside diameter that makes it possible to be inserted on the inside diameter side of the device-side first attachment/detachment part 40A and includes a first circumferential groove 51 provided on the outer circumference on the upstream side and a second circumferential groove 52 provided on the outer circumference on the downstream side. An O-ring 53 is attached to the second circumferential groove 52.

Note that, for the tube-side second attachment/detachment part 50A, some of the elements that are the same as those of the device-side second attachment/detachment part 50B will be referred to by using the same reference characters. Also note that the lock face 49 provided for the operation member 43, the pressing part 45 provided for the operation member 43, and the first circumferential groove 51 of the device-side second attachment/detachment part 50B or the tube-side second attachment/detachment part 50A (to be described later) together form a lock mechanism that locks and unlocks the device-side second attachment/detachment part 50B or the tube-side second attachment/detachment part 50A to and from the tube-side first attachment/detachment part 40B or the device-side first attachment/detachment part 40A.

In other words, in the present embodiment, the lock mechanism is capable of locking and unlocking the tube-side first attachment/detachment part 40B to and from the tube-side second attachment/detachment part 50A or the device-side second attachment/detachment part 50B and is capable of locking and unlocking the device-side first attachment/detachment part 40A to and from the tube-side second attachment/detachment part 50A.

### (Attaching operation)

The tube-side second attachment/detachment part 50A provided for the second attachment/detachment member 80 of the upstream-side tube 101U is attached to the device-side first attachment/detachment part 40A provided for the fluid inlet 25 of the cleaning filter device 1 in the following manner.

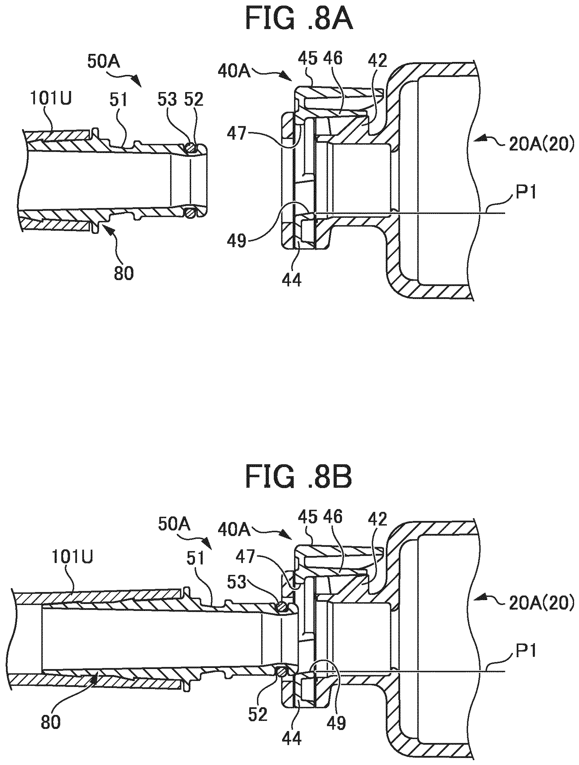

FIGS. 8A-8D are drawings for explaining a procedure of attaching the tube-side second attachment/detachment part 50A provided for the second attachment/detachment member 80 of the upstream-side tube 101U to the device-side first attachment/detachment part 40A of the cleaning filter device 1.

As shown in FIG. 8A, when the tube-side second attachment/detachment part 50A of the second attachment/detachment member 80 has not been plugged into the device-side first attachment/detachment part 40A of the cleaning filter device 1, the urging part 46 abuts against the operation protrusion 42. In that situation, in a bottom section of the drawing, the lock face 49 of the through hole 47 formed in the plug part 44 is positioned at a position P1 indicated in the drawing.

As shown in FIG. 8B, as the tube-side second attachment/detachment part 50A of the second attachment/detachment member 80 is plugged into the device-side first attachment/detachment part 40A of the cleaning filter device 1, the tip end part of the tube-side second attachment/detachment part 50A abuts against the lock face 49 so as to press the lock face 49 downward from the position P1 indicated in the drawing.

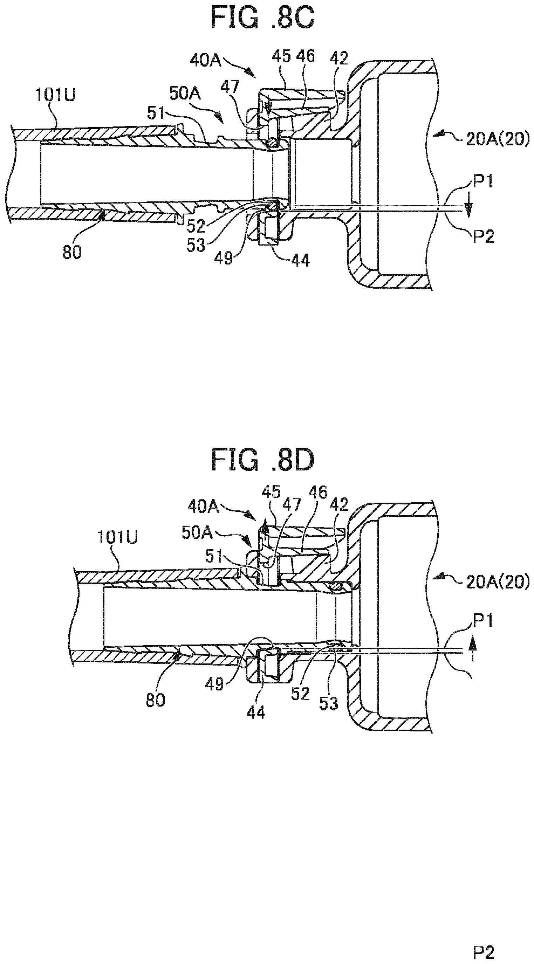

As a result, as shown in FIG. 8C, the tip end of the urging part 46 is abutting against the operation protrusion 42 and is thus unable to move downward; however, the basal end of the urging part 46 moves downward as indicated by the arrow in the drawing, which makes the urging part 46 warped. After that, the position of the lock face 49 moves from the position P1 to a position P2 indicated in the drawing. As a result, it is possible to insert the tube-side second attachment/detachment part 50A of the second attachment/detachment member 80 to the inside of the device-side first attachment/detachment part 40A of the cleaning filter device 1.

As shown in FIG. 8D, when the tube-side second attachment/detachment part 50A of the second attachment/detachment member 80 has been plugged as far as the tip end of the device-side first attachment/detachment part 40A of the cleaning filter device 1, so that the first circumferential groove 51 of the tube-side second attachment/detachment part 50A has reached the position of the plug part 44, the lock face 49 moves from the position P2 to the position P1 indicated in the drawing, due to resilience of the urging part 46.

As a result, because the lock face 49 is fitted in the first circumferential groove 51, the device-side first attachment/detachment part 40A and the tube-side second attachment/detachment part 50A are in a locked state and are prevented from coming off. Thus, the cleaning filter device 1 is connected to the upstream-side tube 101U.

As described above, it is possible to attach the second attachment/detachment member 80 of the upstream-side tube 101U to the device-side first attachment/detachment part 40A of the cleaning filter device 1, with a single action of simply plugging the second attachment/detachment member 80 of the upstream-side tube 101U into the device-side first attachment/detachment part 40A of the cleaning filter device 1. Consequently, it is possible to easily connect the upstream-side tube 101U to the cleaning filter device 1.

### (First attachment/detachment member 90)

Further, as shown in FIG. 2, a downstream-side tube 101D is connected to the device-side second attachment/detachment part 50B positioned on the outlet side of the cleaning filter device 1. To the downstream-side tube 101D, a first attachment/detachment member 90 is attached, while being formed with the tube-side first attachment/detachment part 40B having the same structure as the device-side first attachment/detachment part 40A positioned on the inlet side of the cleaning filter device 1.

As shown in FIG. 2 and FIG. 4, the first attachment/detachment member 90 includes: an insertion part 91 inserted in the downstream-side tube 101D; and the tube-side first attachment/detachment part 40B extending from a tip end of the downstream-side tube 101D toward the outside.

The tube-side first attachment/detachment part 40B has an inside diameter that makes it possible to be inserted on the outside diameter side of the device-side second attachment/detachment part 50B and includes, similarly to the device-side first attachment/detachment part 40A, a slot part 41, an operation protrusion 42, and an operation member 43. Note that, for the tube-side first attachment/detachment part 40B, some of the elements that are the same as those of the device-side first attachment/detachment part 40A will be referred to by using the same reference characters.

### (Attaching operation)

On the downstream side, because the process of attaching the tube-side first attachment/detachment part 40B provided for the first attachment/detachment member 90 of the downstream-side tube 101D to the device-side second attachment/detachment part 50B provided for the fluid outlet 28 of the cleaning filter device 1 is the same as the process of attaching the tube-side second attachment/detachment part 50A provided for the second attachment/detachment member 80 of the upstream-side tube 101U to the device-side first attachment/detachment part 40A provided for the fluid inlet 25 of the cleaning filter device 1, on the upstream side as described above, explanations thereof will be omitted.

It is also possible to attach the tube-side first attachment/detachment part 40B of the downstream-side tube 101D to the device-side second attachment/detachment part 50B of the cleaning filter device 1, with a single action of simply plugging the tube-side first attachment/detachment part 40B of the downstream-side tube 101D into the device-side second attachment/detachment part 50B of the cleaning filter device 1. Consequently, it is possible to easily connect the downstream-side tube 101D to the cleaning filter device 1.

### (Priming)

With reference to FIG. 1 again, at the time of the priming process, the blood circulation circuit 100 is structured as a circulation circuit that does not go through the inside of the patient's body.

At first, a tube 102 having a reservoir needle 103 attached to a tip end thereof is connected to the blood reservoir 3, and the reservoir needle 103 is further connected to a priming-purpose container (not shown) containing saline or the like. When the blood pump 4 is driven, a priming-purpose fluid such as saline is caused to flow into the blood reservoir 3. The fluid caused to flow into the blood reservoir 3 goes through a flow path L1 by being sucked by the blood pump 4 and goes through the artificial lung 5 so as to be separated into a flow path L2 and a flow path L3 running in two directions. The fluid flowing through one of the flow paths, namely L2, enters the blood filter device 6. The fluid flowing through the other flow path L3 is further separated into a flow path L4 and a flow path L5 running in two directions. The fluid in the flow path L4 enters the blood filter device 6 and merges with the fluid in the flow path L2 so as to flow through the flow path L6 and to return to the blood reservoir 3. The fluid in the flow path L5 is further separated into a flow path L7 and a flow path L8 running in two directions. The fluid in the flow path L7 flows through the cleaning filter device 1.

In this situation, in the cleaning filter device 1, unwanted substances in the blood circulation circuit 100 flowing together with the fluid are eliminated. The fluid from which the unwanted substances have been eliminated is separated into a flow path L9 and a flow path L10 and subsequently merged together, so as to flow through the flow path L7 again, to merge with the fluid in the flow path L8, to flow through the flow path L5, and to flow into the blood reservoir 3. Any air present in the blood circulation circuit 100 also flows together with the fluid and flows into the blood reservoir 3.

As shown in FIG. 5, when the first container 20A is linked to the second container 20B, the O-ring 62 is pressed by the end face 23a of the first cylinder part 23 and the inner face 27a of the second lateral end part. Thus, the interior space formed by the first container 20A and the second container 20B is kept airtight (fluid-tight) from the exterior space.

In this situation, when the priming-purpose fluid is caused to flow, the fluid flows as indicated by the arrows in the drawing. In this situation, air bubbles 70 might have a possibility of remaining in small gaps formed between the O-ring 62 and the ridge part 60.

However, in the embodiment, as shown in FIG. 6, the slits 61 are formed in the ridge part 60. Accordingly, as indicated with the arrows in FIG. 6, a part of the fluid that has flowed to the inner face side of the second container 20B is able to go through the slits 61 and to flow through the gaps formed between the O-ring 62 and the ridge part 60. Consequently, the air bubbles 70 between the O-ring 62 and the ridge part 60 also flow out together with the fluid so as to be able to flow out to the blood reservoir 3.

After that, when the priming is completed, the blood pump 4 stops being driven.

### (Removing the cleaning filter device 1)

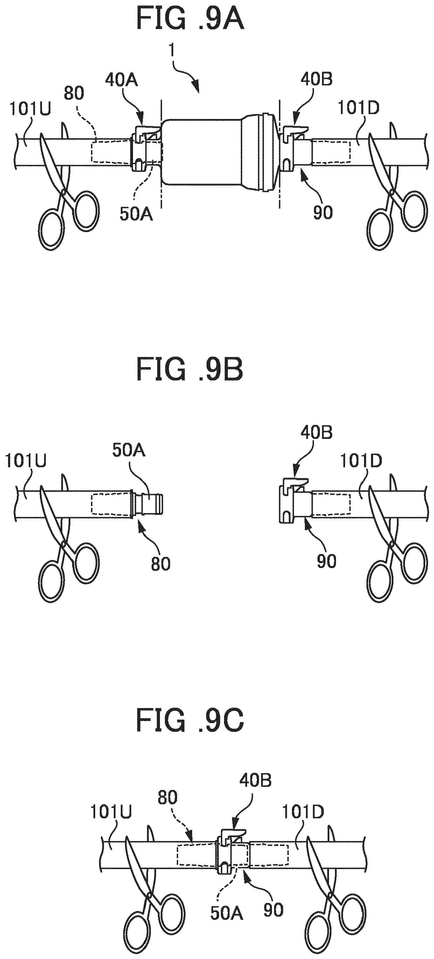

FIGS. 9A-9C are drawings for explaining a procedure of removing the cleaning filter device 1. After the priming is completed, as shown in FIG. 9A, at first, an upstream part and a downstream part on either side of the cleaning filter device 1 are clamped. After that, on the upstream side, the tube-side second attachment/detachment part 50A of the upstream-side tube 101U is detached from the device-side first attachment/detachment part 40A of the cleaning filter device 1.

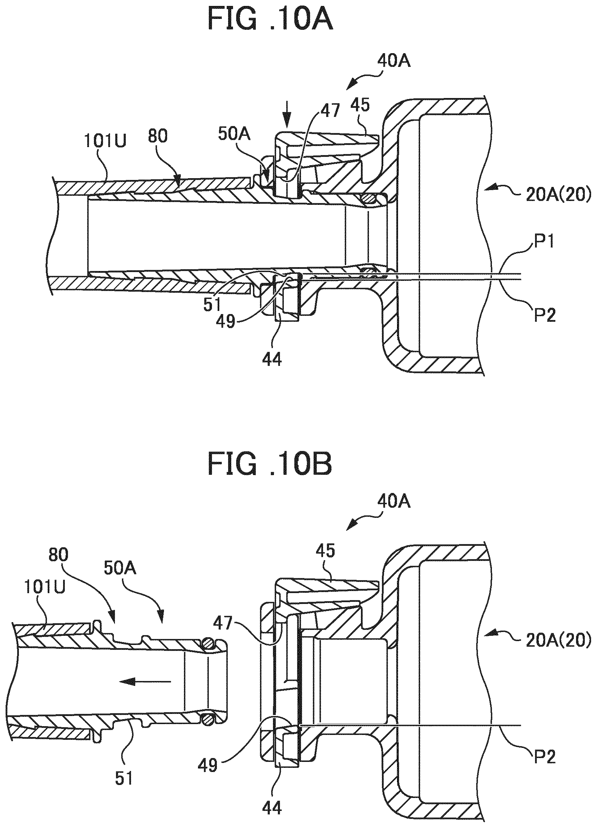

FIGS. 10A-10B are drawings for explaining a procedure of detaching the tube-side second attachment/detachment part 50A from the device-side first attachment/detachment part 40A.

At first, when the pressing part 45 is pressed in the direction indicated by the arrow in FIG. 10A, the plug part 44 moves toward the bottom of the drawing. As a result, the lock face 49 of the through hole 47 formed in the plug part 44 leaves the bottom face of the first circumferential groove 51, so as to achieve an unlocked state in which the device-side first attachment/detachment part 40A is unlocked from the tube-side second attachment/detachment part 50A. As a result, as shown in FIG. 10B, it is possible to pull the tube-side second attachment/detachment part 50A out of the device-side first attachment/detachment part 40A.

As described above, it is possible to detach the tube-side second attachment/detachment part 50A of the second attachment/detachment member 80 of the upstream-side tube 101U from the device-side first attachment/detachment part 40A of the cleaning filter device 1, with a single action of simply pressing the pressing part 45 and pulling the second attachment/detachment member 80 of the upstream-side tube 101U from the device-side first attachment/detachment part 40A of the cleaning filter device 1. Consequently, it is possible to easily detach the upstream-side tube 101U from the cleaning filter device 1.

Further, similarly to detaching the second attachment/detachment member 80 of the upstream-side tube 101U from the device-side first attachment/detachment part 40A of the cleaning filter device 1, it is possible to detach the tube-side first attachment/detachment part 40B of the downstream-side tube 101D from the device-side second attachment/detachment part 50B of the cleaning filter device 1, with an single action of simply pressing the pressing part 45 and pulling the tube-side first attachment/detachment part 40B of the downstream-side tube 101D from the device-side second attachment/detachment part 50B of the cleaning filter device 1. Consequently, it is possible to easily detach the downstream-side tube 101D from the cleaning filter device 1.

Further, as shown in FIG. 9B, the cleaning filter device 1 that has been detached from the tube-side second attachment/detachment part 50A of the second attachment/detachment member 80 inserted in the upstream-side tube 101U and from the tube-side first attachment/detachment part 40B of the first attachment/detachment member 90 inserted in the downstream-side tube 101D is removed from the blood circulation circuit 100.

Subsequently, as shown in FIG. 9C, the tube-side second attachment/detachment part 50A of the second attachment/detachment member 80 of the upstream-side tube 101U is connected to the tube-side first attachment/detachment part 40B of the downstream-side tube 101D. In this situation, as described above, it is possible to connect the tube-side second attachment/detachment part 50A to the tube-side first attachment/detachment part 40B with the single action. Consequently, it is possible to easily connect the upstream-side tube 101U to the downstream-side tube 101D.

Note that after the tubes are connected to each other, when the flow path L9 and the flow path L10 shown in FIG. 1 may each be cut partway so as to connect a catheter in the cut part, the blood circulation circuit 100 becomes an artificial cardiopulmonary system from which the unwanted substances have been eliminated and in which no air bubbles remain.

Further, when the fluid processing device is an artificial lung, the fluid processing device may become clogged during use of the fluid processing device. In that situation, it is possible to replace the clogged fluid processing device or to replace the entire blood circuit including not only the artificial lung but also the fluid processing device.

Also in that situation, it is possible to easily remove the clogged fluid processing device from between the upstream-side tube 101U and the downstream-side tube 101D and to attach a different fluid processing device. In addition, an advantageous effect is achieved where it is possible to reduce the cost of replacing the entirety of the extracorporeal circulation system including circuits.

Some preferred embodiments of the present invention have been described in the foregoing. However, the present invention is not limited to the embodiments described above and may be carried out in various modes.

For example, the structure of the lock mechanism is not limited to the lock face 49 and the operation member 43 as described in the embodiment and may have other structures as long as the attaching/detaching process is possible with a single action, in an extracorporeal circulation circuit including a first tube having a tube-side first attachment/detachment part provided at an end portion thereof; a second tube having a tube-side second attachment/detachment part provided at an end portion thereof, the tube-side second attachment/detachment part being attachable to and detachable from the tube-side first attachment/detachment part; and a fluid processing device including a pair of ports (a fluid inlet and a fluid outlet) and being provided, at one of the ports (one of the fluid inlet and the fluid outlet), with a device-side first attachment/detachment part and provided, at the other port (the other of the fluid inlet and the fluid outlet), with a device-side second attachment/detachment part, in which fluid-tight connection and disconnection is achievable between the device-side second attachment/detachment part and the tube-side first attachment/detachment part and between the device-side first attachment/detachment part and the tube-side second attachment/detachment part, and in a state in which the device-side second attachment/detachment part is disconnected from the tube-side first attachment/detachment part, and the device-side first attachment/detachment part is disconnected from the tube-side second attachment/detachment part, fluid-tight connection and disconnection is achievable between the tube-side first attachment/detachment part and the tube-side second attachment/detachment part.

In the embodiment, the device-side first attachment/detachment part 40A and the tube-side first attachment/detachment part 40B are provided at the fluid inlet 25 of the cleaning filter device 1 and at the end portion of the downstream-side tube 101D, whereas the tube-side second attachment/detachment part 50A and the device-side second attachment/detachment part 50B are provided at the end portion of the upstream-side tube 101U and at the fluid outlet 28 of the cleaning filter device 1.

However, possible embodiments are not limited to this example. The tube-side second attachment/detachment part 50A and the device-side second attachment/detachment part 50B may be provided at the end portion of the downstream-side tube 101D and the fluid inlet 25 of the cleaning filter device 1, whereas the device-side first attachment/detachment part 40A and the tube-side first attachment/detachment part 40B may be provided at the fluid outlet 28 of the cleaning filter device 1 and the end portion of the upstream-side tube 101U.

### EXPLANATION OF REFERENCE NUMERALS

1: Cleaning Filter Device (Fluid Processing Device)
5: Artificial Lung
10: Filter Member
10A: Supported Spacer
10B: Hollow Yarn Wound Body
11: Urethane Resin
12: Core Member
13: Spacer
14: Hollow Yarn
20: Container
20A: First Container
20B: Second Container
23: First Cylinder Part
23a: End face
24: First End Face Part
25: Fluid Inlet (First Fluid Inlet/Outlet)
26: Second Cylinder Part
27: Second End Face Part
27a: Inner face
28: Fluid Outlet (Second Fluid Inlet/Outlet)
30A: First Link Part
30B: Second Link Part
32: Engagement Protrusion
33: Ring-Shaped Protrusion
34: Slope
37: Engagement Hole
38: Tip End Part
40A: Device-Side First Attachment/Detachment Part
40B: Tube-Side First Attachment/Detachment Part
41: Slot Part
42: Operation Protrusion
43: Operation Member (Lock Mechanism)
44: Plug Part (Lock Mechanism)
45: Pressing Part
46: Urging Part
47: Through Hole
49: Lock Face (Lock Mechanism)
50A: Tube-Side Second Attachment/Detachment Part
50B: Device-Side Second Attachment/Detachment Part
51: First Circumferential Groove (Lock Mechanism)
60: Ridge Part
61: Slit
62: O-Ring
70: Air Bubble
80: Second Attachment/Detachment Member
81: Insertion Part
82: Abutment Part
90: First Attachment/Detachment Member
91: Insertion Part
100: Blood Circulation Circuit (Extracorporeal Circulation Circuit)
101U: Upstream-Side Tube (Second Tube)
101D: Downstream-Side Tube (First Tube)

## Claims

1. An extracorporeal circulation circuit comprising:
a first tube having a tube-side first attachment/detachment part provided at an end portion thereof;
a second tube having a tube-side second attachment/detachment part provided at an end portion thereof, the tube-side second attachment/detachment part being attachable to and detachable from the tube-side first attachment/detachment part; and
a fluid processing device having a fluid inlet and a fluid outlet, fesand including a device-side first attachment/detachment part that is provided at one of the fluid inlet and the fluid outlet and that is attachable to and detachable from the tube-side second attachment/detachment part, and a device-side second attachment/detachment part that is provided at an other of the fluid inlet and the fluid outlet and that is attachable to and detachable from the tube-side first attachment/detachment part.

2. The extracorporeal circulation circuit according to claim 1, further comprising:
a lock mechanism capable of locking and unlocking the tube-side first attachment/detachment part or the device-side first attachment/detachment part to and from the tube-side second attachment/detachment part or the device-side second attachment/detachment part.

3. The extracorporeal circulation circuit according to claim 1 or 2, wherein
the fluid processing device is a filter device.

4. The extracorporeal circulation circuit according to any one of claims 1 to 3, wherein
the fluid processing device is disposed at a time of priming.

5. A connection structure comprising:
a tube-side first attachment/detachment part provided at an end portion of a first tube;
a tube-side second attachment/detachment part that is provided at an end portion of a second tube and that is attachable to and detachable from the tube-side first attachment/detachment part;
a device-side first attachment/detachment part that is provided at one of a fluid inlet and a fluid outlet of a fluid processing device and that is attachable to and detachable from the tube-side second attachment/detachment part; and
a device-side second attachment/detachment part that is provided at an other of the fluid inlet and the fluid outlet of the fluid processing device and that is attachable to and detachable from the tube-side first attachment/detachment part.

6. The connection structure according to claim 5, further comprising:
a lock mechanism capable of locking and unlocking the tube-side first attachment/detachment part or the device-side first attachment/detachment part to and from the tube-side second attachment/detachment part or the device-side second attachment/detachment part.

7. A processing method for a fluid processing device in an extracorporeal circulation circuit including:
a first tube having a tube-side first attachment/detachment part provided at an end portion thereof; a second tube having a tube-side second attachment/detachment part provided at an end portion thereof, the tube-side second attachment/detachment part being attachable to and detachable from the tube-side first attachment/detachment part; and a fluid processing device having, at a fluid inlet, a device-side second attachment/detachment part attachable to and detachable from the tube-side first attachment/detachment part and having, at a fluid outlet, a device-side first attachment/detachment part attachable to and detachable from the tube-side second attachment/detachment part,
the processing method comprising:
a fluid circulation step that is performed in a state in which the device-side second attachment/detachment part is connected to the tube-side first attachment/detachment part and the device-side first attachment/detachment part is connected to the tube-side second attachment/detachment part and includes causing a fluid to flow into the extracorporeal circulation circuit to thereby cause the fluid to flow into the fluid processing device through the first tube and to flow out to the second tube;
a detachment step that is performed after the fluid circulation step and includes detaching the tube-side first attachment/detachment part from the device-side second attachment/detachment part and detaching the tube-side second attachment/detachment part from the device-side first attachment/detachment part; and
a step of connecting the tube-side first attachment/detachment part to the tube-side second attachment/detachment part, or
a step of replacing the fluid processing device with a new fluid processing device.
